# EUROPEAN PATENT APPLICATION

(11) **EP 2 631 290 A1**
(43) Date of publication of application: **28.08.2013**
(21) Application number: 11834485.2
(22) Date of filing: 21.10.2011
(51) Int. Cl.: C12N 15/09

(54) **VIRUS VECTOR FOR PRIME/BOOST VACCINES, WHICH COMPRISES VACCINIA VIRUS VECTOR AND SENDAI VIRUS VECTOR**

(30) Priority: 22.10.2010 JP 2010237954
(71) Applicant: National University Corporation Hokkaido University, Sapporo-shi, Hokkaido 060-0808 (JP); Sapporo Medical University, Hokkaido 060-0061 (JP); Dnavec Corporation, Ibaraki 300-2611 (JP)
(72) Inventor: SHIDA Hisatoshi, Hokkaido 0600808 (JP); SOBUE Tomoyoshi, Hokkaido 0600808 (JP); KATO Kazunori, Kokkaido 0600061 (JP); INOUE, Makoto, Tsukuba-shi Ibaraki 300-2611 (JP); HASEGAWA, Mamoru, Tsukuba-shi Ibaraki 300-2611 (JP)
(74) Representative: Wasner, Marita
(86) International application number: PCT/JP2011/074349
(87) International publication number: WO 2012/053646

(57) **Abstract**

To provide a set of virus vectors which can be used for producing a prime/boost vaccine that can activate both cellular immunity and humoral immunity and is effective on infections by pathogenic microorganisms and malignant tumors which are generally believed to be difficult to be treated by vaccine therapy.

[Solution]

Provided is a set of virus vectors for prime/boost vaccines, comprising the following virus vector (a) and virus vector (b): (a) a vaccinia virus vector which carries a gene encoding an immunogenic polypeptide in such a manner that the gene can be expressed; and (b) a Sendal virus vector which carries the gene encoding the immunogenic polypeptide in such a manner that the gene can be expressed.

SEQUENCE LISTING

<110>  National University Corporation Hokkaido University
       SAPPORO MEDICAL UNIVERSITY
       DNAVEC CORPORATION

<120>  Prime/Boost vaccine composed of vaccinia virus and hemagglutinating
virus of japan

<130>  PCT0493HD

<150>  JP 2010-237954
<151>  2010-10-22

<160>  26

<170>  PatentIn version 3.1

<210>  1
<211>  7
<212>  DNA
<213>  Human immunodeficiency virus

<400>  1
tttttat                                                                7


<210>  2
<211>  7
<212>  DNA
<213>  Artificial

<220>
<223>  mutation

<400>  2
tttctat                                                                7


<210>  3
<211>  7
<212>  DNA
<213>  Human immunodeficiency virus

<400>  3
tttttct                                                                7


<210>  4
<211>  7
<212>  DNA
<213>  Artificial

<220>
<223>  mutation

<400>  4
ttcttct                                                                7


<210>  5
<211>  7
<212>  DNA

<213>  Human immunodeficiency virus

<400>  5
tttttct                                                                 7


<210>  6
<211>  7
<212>  DNA
<213>  Artificial

<220>
<223>  mutation

<400>  6
tttctct                                                                 7


<210>  7
<211>  36
<212>  DNA
<213>  Artificial

<220>
<223>  primer

<400>  7
tttcggaccg ccaccatgag agtgaagggg atcagg                                36


<210>  8
<211>  34
<212>  DNA
<213>  Artificial

<220>
<223>  primer

<400>  8
ataggccggc cttatagcaa agccctttcc aagc                                  34


<210>  9
<211>  36
<212>  DNA
<213>  Artificial

<220>
<223>  primer

<400>  9
agtggatccg ccagcatgat cgaaacatac aaccaa                                36


<210>  10
<211>  33
<212>  DNA
<213>  Artificial

<220>
<223>  primer

<400>  10

agacccgagt cagagtttga gtaagccaaa gga                                    33

<210>  11
<211>  39
<212>  DNA
<213>  Artificial

<220>
<223>  primer

<400>  11
ctagaattcg ccaccatgag agtgaagggg atcaggaag                             39

<210>  12
<211>  33
<212>  DNA
<213>  Artificial

<220>
<223>  primer

<400>  12
cgtgagctct tatagcaaag ccctttccaa gcc                                   33

<210>  13
<211>  39
<212>  DNA
<213>  Artificial

<220>
<223>  primer

<400>  13
ctagagctcg ccaccatata ctatatagta ataccaata                             39

<210>  14
<211>  32
<212>  DNA
<213>  Artificial

<220>
<223>  primer

<400>  14
gtacccgggt cagagtttga gtaagccaaa gg                                    32

<210>  15
<211>  39
<212>  DNA
<213>  Artificial

<220>
<223>  primer

<400>  15
tttcggaccg ccaccatgag agtgaagggg atcaggaag                             39

<210>  16
<211>  35
<212>  DNA
<213>  Artificial

<220>
<223>  primer

<400>  16
agaggccggc ctcagagttt gagtaagcca aagga                                35


<210>  17
<211>  32
<212>  DNA
<213>  Artificial

<220>
<223>  primer

<400>  17
aaacccgggc atgatcgaaa catacaacca aa                                   32


<210>  18
<211>  31
<212>  DNA
<213>  Artificial

<220>
<223>  primer

<400>  18
ccatctagat cctcagagtt tgagtaagcc a                                    31


<210>  19
<211>  42
<212>  DNA
<213>  Artificial

<220>
<223>  primer

<400>  19
ccatcgtctt cactcactcc tcaggagggg atccagaaat tg                        42


<210>  20
<211>  83
<212>  DNA
<213>  Artificial

<220>
<223>  primer

<400>  20
gaataacact ttaaaacaga tagttgagaa gctccgcgag cagttcaaca acaagaccat     60

cgtcttcact cactcctcag gag                                             83


<210>  21

<211>  42
<212>  DNA
<213>  Artificial

<220>
<223>  primer

<400>  21
gtgaagatcg aaccattagg agtagcaccc accaaggcaa ag                          42


<210>  22
<211>  71
<212>  DNA
<213>  Artificial

<220>
<223>  primer

<400>  22
gagacatgag ggacaattgg agaagtgagc tctacaagta caaggtcgtg aagatcgaac      60

cattaggagt a                                                            71


<210>  23
<211>  46
<212>  DNA
<213>  Artificial

<220>
<223>  primer

<400>  23
cgcatcgtgt tctctgtact ttctatagtg aatagagtta ggcagg                      46


<210>  24
<211>  98
<212>  DNA
<213>  Artificial

<220>
<223>  primer

<400>  24
gtttgacata acaaaatggc tgtggtacat caagatcttc atcatgatcg tgggaggcct      60

gatcggtctc cgcatcgtgt tctctgtact ttctatag                               98


<210>  25
<211>  33
<212>  DNA
<213>  Artificial

<220>
<223>  primer

<400>  25
ctagaattcg gcatctccta tggcaggaag aag                                    33

<210>  26
<211>  32
<212>  DNA
<213>  Artificial

<220>
<223>  primer

<400>  26
cgtgaattca cccatcttat agcaaagccc tt                                32

## Description

### Technical Field

The present invention relates to a set of virus vectors for a prime/boost vaccine, specifically, a set of virus vectors for a prime/boost vaccine which can be used in a prime/boost vaccine that can activate both cellular immunity and humoral immunity.

### Background Art

Human beings are exposed to risk of infection with viruses, bacteria, fungi, or other various organisms and of infectious diseases caused thereby. One measure for overcoming such infectious diseases is vaccine administration. Vaccines are roughly classified into live vaccines using pathogenic microorganisms themselves, such as attenuated bacteria or viruses and inactivated vaccines using pathogenic microorganisms killed by, for example, chemical treatment or immunogenic portions thereof.

Vaccines are desired to be capable of activating both cellular immunity and humoral immunity from the viewpoint of providing strong immunity. Live vaccines can activate the both and are therefore effective in provision of strong acquired immunity and long duration of immunity. However, though they are attenuated, since pathogenic microorganisms themselves are administered in vivo, it is difficult to completely deny the possibility of, for example, side reaction by infection with a pathogenic microorganism or virulence increased by reversion (back mutation or atavism) of the attenuated pathogenic microorganism.

Accordingly, inactivated vaccines are used for pathogenic microorganisms having possibility of causing significantly serious disease to a subject once infected therewith, such as human immunodeficiency virus (HIV) and human hepatitis virus (HCV). In particular, virus vector vaccines containing genes encoding antigen proteins derived from pathogenic microorganisms, incorporated into genomes of infectious viruses having high safety to human by genetic recombination, are used.

These virus vector vaccines need virus vectors showing high safety in vivo and high ability of expressing recombinant genes. Examples of the usable virus vector include viruses not capable of proliferating in mammals and viruses having genomes modified such that the viruses can infect but cannot produce descendant viruses in the host. Specific examples of the usable virus include canarypox virus, vaccinia virus MVA (modified vaccinia virus Ankara), vaccinia virus strain LC16m8, vaccinia virus strain m8ΔB5R having deletion of the B5R gene of vaccinia virus strain LC16m8 and not producing any B5R gene product having a normal function (Patent Literature 1), adenovirus being deficient in E1 and E3 genes of vector subtype 5, and Sendai virus.

In addition, many virus vector vaccines have been developed, e.g., in addition to a human hepatitis B vaccine using vaccinia virus strain LC16m8 as the vector (Non Patent Literature 1), HIV vaccines using various virus vectors (e.g., adenovirus vectors, Sendai virus vectors, vaccinia virus vectors, and canarypox virus vectors) containing genes of HIV-1 structural proteins represented by a HIV vaccine having a HIV envelope protein gp160 (GenBank No. U21135) incorporated into vaccinia virus strain LC16m8 (Patent Literature 2) and an HIV vaccine having a HIV gag protein incorporated into a Sendai virus vector (Patent Literature 3).

The mainstream of vaccination, on the other hand, has been being transferred from a method using only one type of vaccine to a method using a prime/boost vaccine composed of two or more types of vaccines. Many prime/boost vaccines composed of plasmids (DNA vaccines) containing antigen protein genes and various virus vectors have been tried, and also a prime/boost HIV vaccine using an rBCG/HIV-1 gag E vaccine having a HIV-1 gag gene incorporated into BCG having high safety for priming and a vaccinia DIs/HIV-1 gag E vaccine having a HIV-1 gag protein gene incorporated into attenuated vaccinia virus not proliferating in human bodies, vaccinia virus strain DIs, for boosting (Patent Literature 4) and a prime/boost HIV vaccine composed of a canarypox virus vector and HIV envelope protein gp120 (Non Patent Literature 2) have been developed.

Meanwhile, as CD40 ligand (CD40L) expressed in immune cells such as stimulated CD4 positive T cells and stimulated CD8 positive T cells is a factor of stimulating dendritic cells, methods of activating immunity with this factor have been reported. For example, a method of activating immunity through administration of a soluble CD40L protein, immunotherapy of malignant tumors with dendritic cells stimulated by introduction of a CD40L expression vector, a method of activating immunity by administration of a mixture of a soluble CD40L protein and a plasmid or a nonproliferative vaccinia virus vector (Non Patent Literature 3), and a method of modifying immune reactivity of recombinant cells by expressing CD40 ligand (CD40L) or its non-cleavage mutant CD40Lm in the cells (Patent Literature 5) have been reported.

### Citation List

### Patent Literature

Patent Literature 1
   International Publication No. WO2005/054451
Patent Literature 2
   Japanese Patent Laid-Open No. 2003-321391
Patent Literature 3
   International Publication No. WO2001/072340
Patent Literature 4
   Japanese Patent Laid-Open No. 2006-149234
Patent Literature 5
   International Publication No. WO2005/100558
Non Patent Literature

Non Patent Literature 1
   So Hashizume, "Foundation of novel attenuated vaccinia strain LC16m8", Rinsho to Virus, vol. 3, No. 3, p. 229, 1975
Non Patent Literature 2
   Perks-Ngarm S., et al., N. Engl. J. Med., vol. 361, pp. 2209-2220, 2009
Non Patent Literature 3
   C.E. Gomez, et al., Vaccine, vol. 27, pp. 3165-3174, 2009

### Summary of Invention

### Technical Problem

However, though HIV vaccines of HIV-1 structural protein genes incorporated into various virus vectors activate cellular immunity, the activation of humoral immunity is weak. In addition, clinical study has shown that HIV vaccines using adenovirus vectors for activating cellular immunity do not have an effect of inhibiting infection by HIV-1 (Science, vol. 321, p. 530, 2008). Furthermore, though the method of activating immunity by administration of a mixture of a soluble CD40L protein and a plasmid or a nonproliferative vaccinia virus vector described in Non Patent Literature 3 activates the cellular immunity, the effect of activating humoral immunity is restrictive.

Meanwhile, conventional prime/boost vaccines composed of plasmids and various virus vectors activated cellular immunity, but the antibody-inducing ability was weak. In clinical study, the prime/boost HIV vaccine composed of a canarypox virus vector and a HIV envelope protein gp120 described in Non Patent Literature 2 was shown to reduce the infection rate of HIV-1 by a small degree (30%), but its infection-inhibiting effect is insufficient.

That is, no vector vaccine or prime/boost vaccine that activates both humoral immunity and cellular immunity, which can be decisive for serious diseases such as HIV and HCV, has been accomplished yet. In particular, it is believed that activation of both humoral immunity and cellular immunity is indispensable for inhibiting infection by HIV, but the neutralizing antibody titer produced by the existing vector vaccine for HIV is low and is not an acceptable level for practical use. Thus, there is a demand for developing vector vaccines that can induce both humoral immunity and cellular immunity.

Furthermore, the immunostimulation effect of a prime/boost vaccine varies depending on, for example, the combination of various vector vaccines, the order of vaccines, i.e., which is for priming and which is for boosting, the type of the immunogenic protein, and regulation of expression amount. Accordingly, the situation is still that the selection and determination of these factors, i.e., vaccine designing, must be performed through trial and error.

Furthermore, in addition to infectious diseases such as viral infection, vaccines against tumor antigens, so-called cancer vaccines, are being developed to be used in therapy of malignant tumors, which are one of main causes of human death. The cancer vaccine therapy aims at treating a malignant tumor by specifying an antigenic substance being specifically or significantly expressed in malignant tumor tissue or tumor cells and enhancing the immunity of a patient itself against this antigenic substance. It is practically impossible to use a malignant tumor itself as a live vaccine. Accordingly, there is a demand for developing inactivated vaccines, specifically, vector vaccines that can express tumor antigens in vivo, in particular, prime/boost vaccines, against malignant tumors.

It is an objective of the present invention to provide a set of virus vectors that can be used for producing prime/boost vaccines capable of activating both cellular immunity and humoral immunity and being effective for infectious diseases caused by pathogenic microorganisms and malignant tumors, of which the vaccine therapy is generally recognized to be difficult.

### Solution to Problem

The present inventors have found that both cellular immunity and humoral immunity can be activated by designing vaccines for producing prime/boost vaccines against pathogenic microorganisms such that an antigen protein expressing vaccinia virus vector is used for priming and an antigen protein expressing Sendai virus vector is used for boosting and that the immunostimulation effect is enhanced by expressing CD40Lm together with the antigen protein in the priming, and the inventors have accomplished the following invention:

(1) A set of virus vectors for a prime/boost vaccine, comprising the following virus vector (a) and virus vector (b):
(a) a vaccinia virus vector expressively carrying a gene encoding a polypeptide having immunogenicity; and
(b) a Sendai virus vector expressively carrying a gene encoding a polypeptide having the immunogenicity;

(2) The set of virus vectors for a prime/boost vaccine according to (1), further comprising the following virus vector (c):
(c) a vaccinia virus vector expressively carrying a gene encoding a CD40 ligand non-cleavage mutant;

(3) The set of virus vectors for a prime/boost vaccine according to (1), wherein the vaccinia virus vector expressively carrying a gene encoding a polypeptide having immunogenicity is a vaccinia virus vector expressively carrying a gene encoding a polypeptide having immunogenicity and a gene encoding a CD40 ligand non-cleavage mutant;

(4) The set of virus vectors for a prime/boost vaccine according to any one of (1) to (3), wherein the virus vector (a) or the virus vector (a) and the virus vector(c) are for priming; and the virus vector (b) is for boosting;

(5) The set of virus vectors for a prime/boost vaccine according to any one of (1) to (4), wherein the vaccinia virus vector is a vaccinia virus strain LC16, strain LC16m8, or strain Lc16mO and having substitution, addition, insertion, and/or deletion of one or more nucleotides in its B5R gene not to produce any B5R gene product having a normal function;

(6) The set of virus vectors for a prime/boost vaccine according to any one of (1) to (5), wherein the polypeptide having immunogenicity is an antigen protein of a microorganism pathogenic to human or a partial peptide thereof or is a human tumor antigen protein or its partial peptide; and

(7) The set of virus vectors for a prime/boost vaccine according to (6), wherein the pathogenic microorganism is one selected from the group consisting of human immunodeficiency viruses, influenza viruses, human hepatitis viruses, human papillomaviruses, herpes viruses, flaviviruses, severe acute respiratory syndrome viruses, Japanese encephalitis viruses, measles viruses, rubella viruses, mumps viruses, yellow fever viruses, rabies viruses, Ebola viruses, Lassa viruses, polio viruses, St. Louis encephalitis viruses, cholera vibrios, tubercle bacilli, diphtheria bacilli, typhoid bacilli, Whooping cough bacilli, meningococci, tetanus bacilli, mycobacteria, and malaria parasites.

### Advantageous Effects of Invention

The set of virus vectors for a prime/boost vaccine of the present invention can activate not only cellular immunity such as production of cytokines specific to a pathogenic microorganism but also humoral immunity such as production of an antibody specific to the pathogenic microorganism and, therefore, can be used in a prime/boost vaccine. That is, a prime/boost vaccine employing the set of virus vectors for prime/boost vaccine of the present invention can inhibit infection with a pathogenic microorganism, such as HIV, of which the infection cannot be sufficiently inhibited conventionally. Furthermore, prime/boost vaccines effective for prevention or therapy of various infectious diseases and malignant tumors can be produced by appropriately replacing the gene encoding a polypeptide having immunogenicity carried by the set of virus vectors for a prime/boost vaccine of the present invention by that derived from various pathogenic microorganisms or malignant tumors.

### Brief Description of Drawings

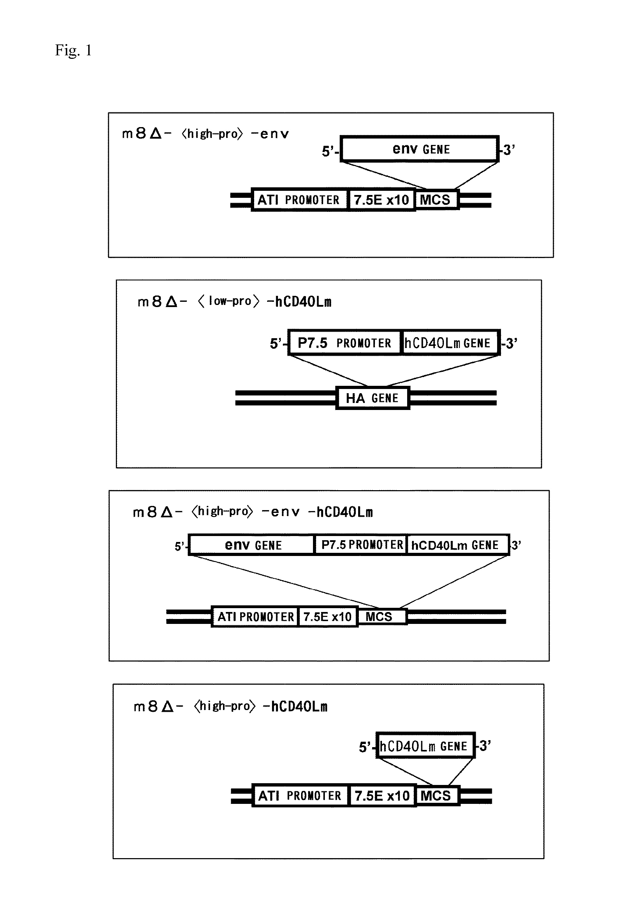

[Figure 1] Figure 1 includes schematic diagrams illustrating the structures of genes and promoters inserted into genomes of m8Δ-<high-pro>-env, m8Δ-<low-pro>-hCD40Lm, m8Δ-<high-pro>-env-hCD40Lm, and m8Δ-<high-pro>-hCD40Lm.

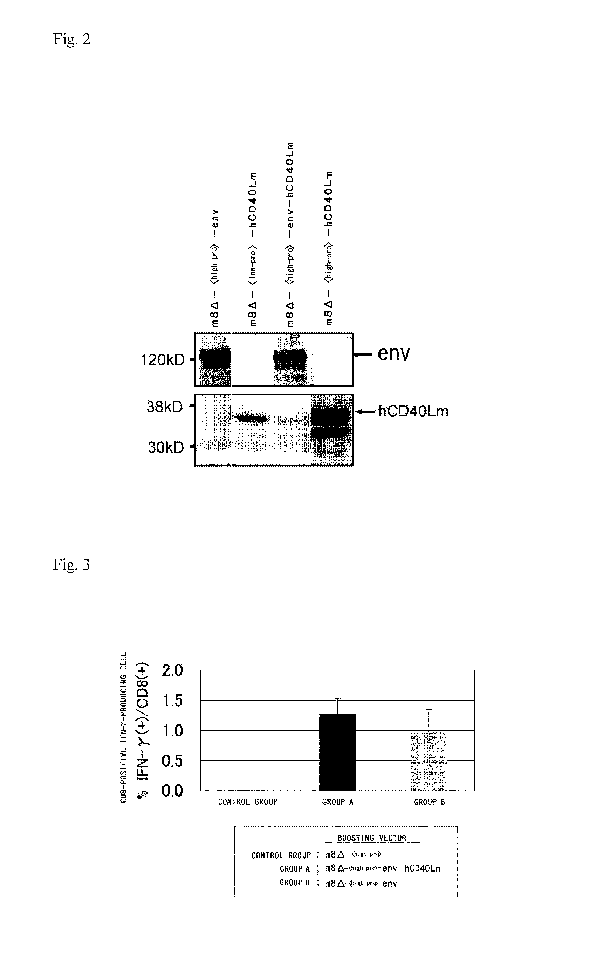

[Figure 2] Figure 2 is a diagram showing the results of Western blotting of detecting expression of env and hCD40Lm in rabbit kidney-derived cells (RK13 cells) infected with m8Δ-<high-pro>-env, m8Δ-<low-pro>-hCD40Lm, m8Δ-<high-pro>-env-hCD40Lm, or m8Δ-<high-pro>-hCD40Lm.
[Figure 3] Figure 3 is a graph showing the results of counting the number of CD8-positive IFN-γ-producing cells in the spleen of mice primed with an antigen protein expressing plasmid (DNA-env) and then boosted with a vaccinia virus vector (m8Δ-<high-pro>: control group); boosted with an antigen protein/CD40Lm coexpressing vaccinia virus vector (m8Δ-<high-pro>-env-hCD40Lm: group A); or boosted with an antigen protein expressing vaccinia virus vector (m8Δ-<high-pro>-env: group B).

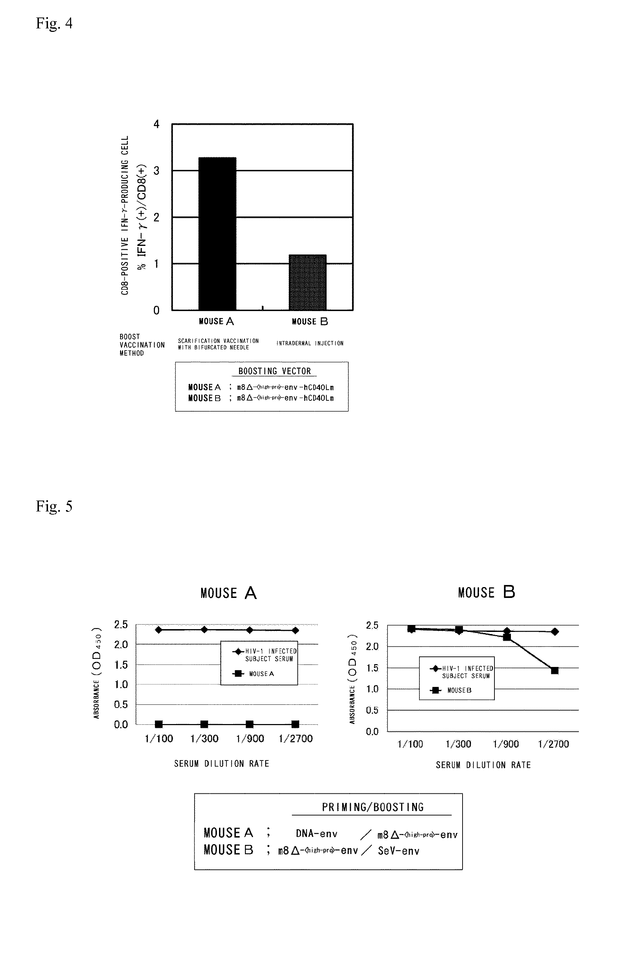

[Figure 4] Figure 4 is a graph showing the results of counting the number of CD8-positive IFN-γ-producing cells in the spleen of mice primed with an antigen protein expressing plasmid (DNA-env) and then boosted with an antigen protein/CD40Lm coexpressing vaccinia virus vector (m8Δ-<high-pro>-env-hCD40Lm) by scarification vaccination using a bifurcated needle (mouse A); or boosted with an antigen protein/CD40Lm coexpressing vaccinia virus vector (m8Δ-<high-pro>-env-hCD40Lm) by intradermal injection (mouse B).
[Figure 5] Figure 5 includes a graph (at the left) showing the measurement results of the binding affinity of an anti-env antibody in the serum of a mouse (mouse A) primed with an antigen protein expressing plasmid (DNA-env) and then boosted with an antigen protein expressing vaccinia virus vector (m8Δ-<high-pro>-env); and a graph (at the right) showing the measurement results of the binding affinity of an anti-env antibody in the serum of a mouse (mouse B) primed with the antigen protein expressing vaccinia virus vector (m8Δ-<high-pro>-env) and then boosted with an antigen protein expressing Sendai virus vector (SeV-env).

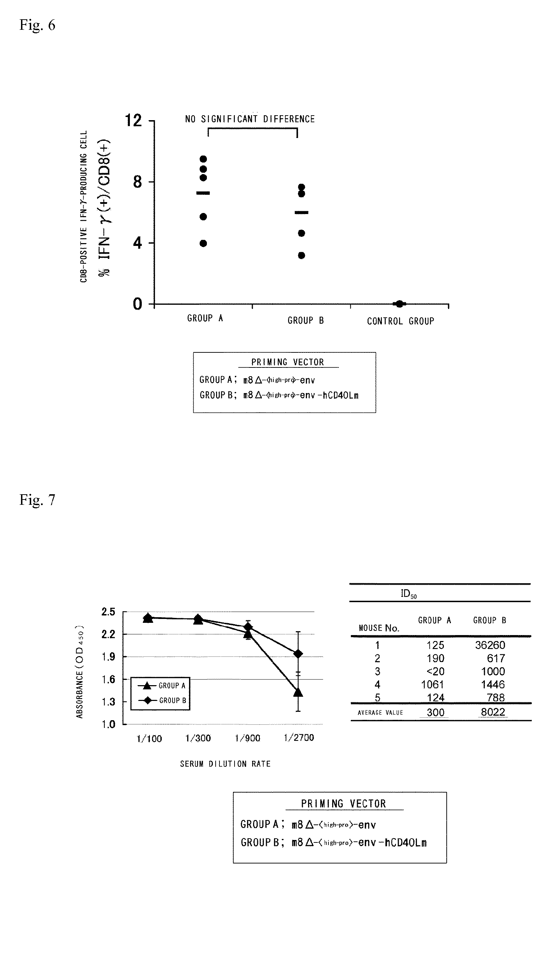

[Figure 6] Figure 6 is a graph showing the results of counting the number of CD8-positive IFN-y-producing cells in the spleen of mice (group A) primed with an antigen protein expressing vaccinia virus vector (m8Δ-<high-pro>-env) and then boosted with an antigen protein expressing Sendai virus vector (SeV-env); mice (group B) primed with an antigen protein/CD40Lm coexpressing vaccinia virus vector (m8Δ-<high-pro>-env-hCD40Lm) and then boosted with an antigen protein expressing Sendai virus vector (SeV-env); and mice (control group) not vaccinated.
[Figure 7] Figure 7 includes a table (at the right) showing the binding affinity of anti-env antibodies in the serum of mice (group A) primed with an antigen protein expressing vaccinia virus vector (m8Δ-<high-pro>-env) and then boosted with an antigen protein expressing Sendai virus vector(SeV-env) and mice (group B) primed with an antigen protein/CD40Lm coexpressing vaccinia virus vector (m8Δ-<high-pro>-env-hCD40Lm) and then boosted with an antigen protein expressing Sendai virus vector (SeV-env); and a graph (at the left) showing the measurement results of neutralizing activity of anti-env antibodies in the serum of mice in group A and group B.

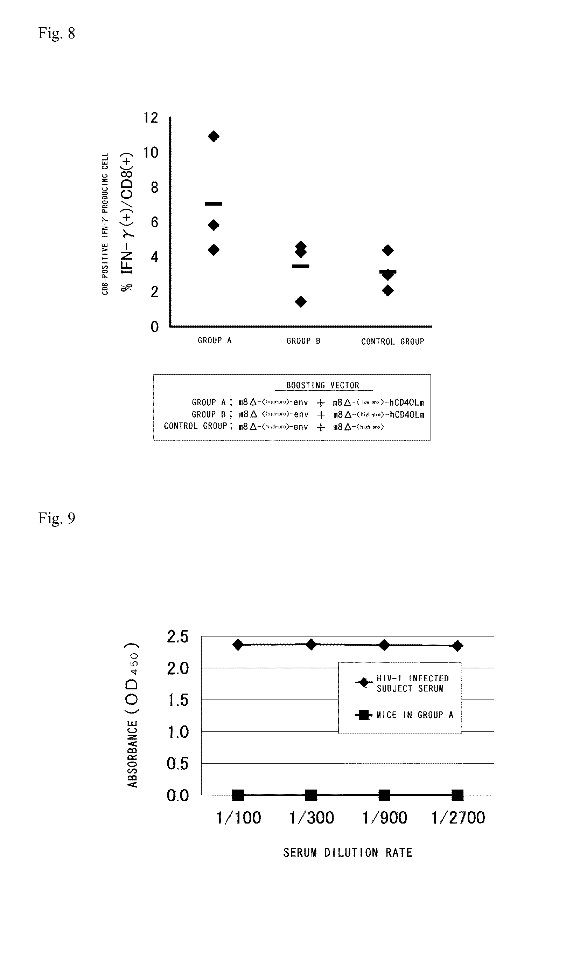

[Figure 8] Figure 8 is a graph showing the results of counting the number of CD8-positive IFN-γ-producing cells in the spleen of mice primed with an antigen protein expressing plasmid (DNA-env) and then boosted with an antigen protein expressing vaccinia virus vector (m8Δ-<high-pro>-env) and a CD40Lm low-expressing vaccinia virus vector (m8Δ-<low-pro>-hCD40Lm) (group A); boosted with an antigen protein expressing vaccinia virus vector (m8Δ-<high-pro>-env) and a CD40Lm high-expressing vaccinia virus vector (m8Δ-<high-pro>-hCD40Lm) (group B); and boosted with an antigen protein expressing vaccinia virus vector (m8Δ-<high-pro>-env) and a vaccinia virus vector (m8Δ-<high-pro>) (control group).
[Figure 9] Figure 9 is a graph showing typical measurement results of the binding affinity of anti-env antibodies in the serum of mice (group A) primed with an antigen protein expressing plasmid (DNA-env) and then boosted with an antigen protein expressing vaccinia virus vector (m8Δ-<high-pro>-env) and a CD40Lm high-expressing vaccinia virus vector (m8Δ-<high-pro>-hCD40Lm).

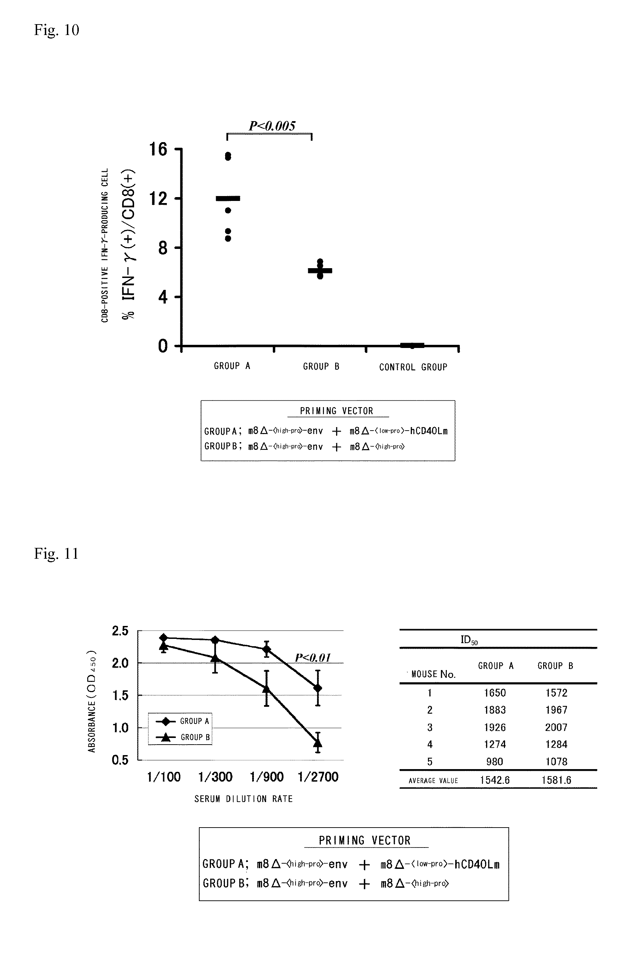

[Figure 10] Figure 10 is a graph showing the results of counting the number of CD8-positive IFN-γ-producing cells in the spleen of mice (group A) primed with an antigen protein expressing vaccinia virus vector (m8Δ-<high-pro>-env) and a CD40Lm low-expressing vaccinia virus vector (m8Δ-<low-pro>-hCD40Lm) and then boosted with an antigen protein expressing Sendai virus vector (SeV-env); mice (group B) primed with an antigen protein expressing vaccinia virus vector (m8Δ-<high-pro>-env) and a vaccinia virus vector (m8Δ-<high-pro>) and then boosted with an antigen protein expressing Sendai virus vector (SeV-env); and mice (control group) not vaccinated.
[Figure 11] Figure 11 includes a table (at the right) showing the binding affinity of anti-env antibodies in the serum of mice (group A) primed with an antigen protein expressing vaccinia virus vector (m8Δ-<high-pro>-env) and a CD40Lm low-expressing vaccinia virus vector (m8Δ-<low-pro>-hCD40Lm) and then boosted with an antigen protein expressing Sendai virus vector (SeV-env) and mice (group B) primed with an antigen protein expressing vaccinia virus vector (m8Δ-<high-pro>-env) and a vaccinia virus vector (m8Δ-<high-pro>) and then boosted with an antigen protein expressing Sendai virus vector (SeV-env); and a graph (at the left) showing the measurement results of neutralizing activity of anti-env antibodies in the serum of mice in groups A and B.

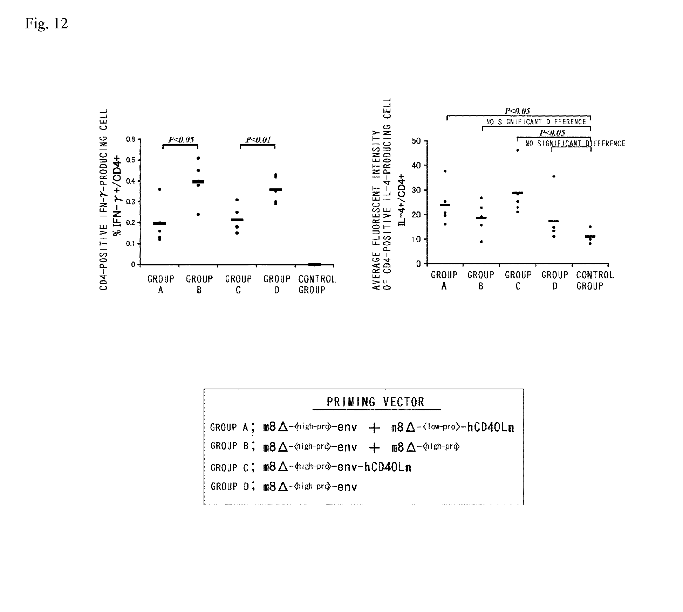

[Figure 12] Figure 12 includes graphs showing the results of counting the number of CD4-positive IFN-γ-producing cells and the average fluorescent intensity of CD4-positive IL-4-producing cells (at the left and the right, respectively) in the spleen of mice (group A) primed with an antigen protein expressing vaccinia virus vector (m8Δ-<high-pro>-env) and a CD40Lm low-expressing vaccinia virus vector (m8Δ-<low-pro>-hCD40Lm) and then boosted with an antigen protein expressing Sendai virus vector (SeV-env); mice (group B) primed with an antigen protein expressing vaccinia virus vector (m8Δ-<high-pro>-env) and a vaccinia virus vector (m8Δ-<high-pro>) and then boosted with an antigen protein expressing Sendai virus vector (SeV-env); mice (group C) primed with an antigen protein/CD40Lm coexpressing vaccinia virus vector (m8Δ-<high-pro>-env-hCD40Lm) and then boosted with an antigen protein expressing Sendai virus vector (SeV-env); mice (group D) primed with an antigen protein expressing vaccinia virus vector (m8Δ-<high-pro>-env) and then boosted with an antigen protein expressing Sendai virus vector (SeV-env); and mice (control group) not vaccinated.

### Description of Embodiments

The set of virus vectors for a prime/boost vaccine according to the present invention will now be described in detail. The set of virus vectors for a prime/boost vaccine according to the present invention comprises a vaccinia virus vector (a) expressively carrying a gene encoding a polypeptide having immunogenicity and a Sendai virus vector (b) expressively carrying a gene encoding a polypeptide having the immunogenicity.

The prime/boost vaccine is composed of two or more types of vaccine including a vaccine used in primary immunization (prime or priming) and a vaccine used in booster immunization (boost or boosting). Usually, the vaccine used in primary immunization and the vaccine used in booster immunization are different from each other.

The set of virus vectors for a prime/boost vaccine according to the present invention includes a vaccinia virus vector expressively carrying a gene encoding a polypeptide having immunogenicity. The vaccinia virus vector is an excellent vector because of its characteristics of inducing a proper immune reaction in human, in addition to its safety. Examples of the vaccinia virus vector that can be used in the present invention include strain LC16, strain LC16m8, strain LC16mO, strain DIs, and strain MVA. In particular, the vaccinia virus vector is preferably any of these strains of which the B5R gene has substitution, addition, insertion, and/or deletion of one or more nucleotides not to produce any B5R gene product having a normal function (Patent Literature 1). No production of any B5R gene product having a normal function can solve the problem of back mutation or so-called atavism, i.e. virulence increased by reversion of the vaccinia virus. Examples of the vaccinia virus vector not producing any B5R gene product having a normal function include strain LC16, m8ΔB5R (strain LC16m8Δ), mOΔB5R (strain LC16mOΔ), m8proB5RdTM, and mOproB5RdTM, which stains have deletion of the B5R gene. In particular, strain LC16, m8ΔB5R (strain LC16m8Δ), and mOΔB5R (strain LC16mOΔ) having deletion of the B5R gene are preferred. The details of the vaccinia virus vector not producing any B5R gene product having a normal function are as described in Patent Literature 1.

Strain LC16m8 used in vaccination has been inoculated to about one hundred thousand infants and about three thousand adults, but no serious adverse effect has been reported. However, the strain LC16m8 is genetically unstable and has a disadvantage of generating a virulent revertant. The present inventors have produced strain LC16m8Δ that does not generate any revertant. The strain LC16m8Δ has excellent immunity induction compared with strain DIs and strain MVA, which are vaccinia virus strains that cannot proliferate (M. Kidokoro, et al., Proc. Natl. Acad. Sci., vol. 102, pp. 4152-4157, 2005; H. Suzuki, et al., Vaccine, vol. 27, pp. 966-971, 2009). It has been reported that the strain LC16m8Δ also prevented monkey from being infected with monkeypox being highly pathogenic (The Japanese Society for Virology, 2006). From the above, the strain LC16m8Δ is expected to be safe to human and to be capable of inducing excellent immunity.

The number of nucleotides substituted, added, inserted, and/or deleted in "substitution, addition, insertion, and/or deletion of one or more nucleotides," in the present invention, is not particularly limited as long as the B5R gene product produced by transcription and translation does not have a normal function, and can be, for example, within 1 to 997, preferably 100 to 997, more preferably 300 to 997, more preferably 500 to 997, and most preferably 700 to 997.

The vaccinia virus vector expressively carrying a gene encoding a polypeptide having immunogenicity in the present invention may be a vaccinia virus vector (coexpression vaccinia virus vector) expressively carrying both a gene encoding a polypeptide having immunogenicity and a gene encoding a CD40 ligand non-cleavage mutant (CD40Lm). The details, such as the function of CD40Lm and the sequence information of the CD40Lm gene, are as described in Patent Literature 4.

The polypeptide having immunogenicity, in the present invention, refers to a polypeptide that can induce immune reaction, cellular immunity and/or humoral immunity, in vivo by administration thereof. Examples of such a polypeptide include antigen proteins of microorganisms pathogenic to human, human tumor antigen proteins, and their partial peptides. In the present invention, the term "activate" is exchangeable for the term "induce" or "stimulate".

The term polypeptide, in the present invention, refers to a compound composed of two or more amino acids bound by peptide bonds, and the number of amino acids constituting the polypeptide is not particularly limited. Examples of the polypeptide include dipeptides each composed of two amino acids, tripeptides each composed of three amino acids, tetrapeptides each composed of four amino acids, oligopeptides each composed of about ten amino acids, and peptides or proteins each composed of 20 or more amino acids.

In the present invention, examples of the microorganism pathogenic to human include human immunodeficiency viruses, influenza viruses, human hepatitis viruses, human papillomaviruses, herpes viruses, flaviviruses, severe acute respiratory syndrome viruses, Japanese encephalitis viruses, measles viruses, rubella viruses, mumps viruses, yellow fever viruses, rabies viruses, Ebola viruses, Lassa viruses, polio viruses, St. Louis encephalitis viruses, cholera vibrios, tubercle bacilli, diphtheria bacilli, typhoid bacilli, Whooping cough bacilli, meningococci, tetanus bacilli, mycobacteria, malaria parasites, group A β-hemolytic streptococci, pneumococci, Streptococcus aureus, Streptococcus epidermidis, enterococci, Listeria, meningococci, gonococci, pathogenic Escherichia coli bacteria, pneumobacilli, Proteus bacilli, Pseuomonas aeruginosa, serratia bacteria, Citrobacter, Acinetobacter, Enterobacter, mycoplasmas, chlamydias, and clostridiums. Examples of the antigen protein of the microorganism pathogenic to human include envelope proteins gp160 and gp120 (env), gyp41, pol protein reverse transcriptase, nef protein, tat protein, gag precursor p55, and p24 protein of human immunodeficiency viruses; hemagglutinin, neuraminidase, and M2 of influenza viruses; envelope proteins E1 and E2 of hepatitis C viruses; and HBs antigen of hepatitis B viruses.

Examples of the human tumor antigen protein include melanocyte tissue-specific protein gp100 (Bakker, et al., J. Exp. Med., vol. 179, p. 1005, 1994); human papillomavirus E6 protein and E7 protein of cervical cancer; melanosome antigens such as MART-1 (Kawakami, et al., Proc. Natl. Acad. Sci., vol. 91, p. 3515, 1994) and tyrosinase (Brichard, et al., J. Exp. Med., vol. 178, p. 489, 1993); HER2/neu (Fisk B., et al., J. Exp. Med., vol. 181, p. 2109, 1995); CEA (Tsang K.Y., et al., J. Natl. Cancer Inst., vol. 87, p. 982, 1995); and PSA (Correale P., et al., J. Natl. Cancer Inst., vol. 89, p. 293, 1997).

In the present invention, the vaccinia virus vector expressively carrying a gene encoding a polypeptide having immunogenicity can be produced by producing a plasmid (transfer vector) linked with the gene encoding a polypeptide having immunogenicity to be introduced and introducing the plasmid into a cell infected with a vaccinia virus to cause homologous recombination in the cell. Alternatively, the vaccinia virus vector can be also produced by directly linking a gene segment, digested with an appropriate restriction enzyme, encoding a polypeptide having immunogenicity to be introduced to the vaccinia virus genome digested with the same enzyme, and introducing the resulting recombinant vaccinia virus genome into a virus-infected cell.

Examples of the plasmid that can be used in production of the vaccinia virus vector expressively carrying a gene encoding a polypeptide having immunogenicity include pSFJ1-10, pSFJ2-16, pMM4, pGS20, pSC11, pMJ601, p2001, pBCB01-3,06, pTKgpt-F1-3s, pTM1, pTM3, pPR34,35, pgpt-ATA18-2, pHES1-3, pJW322, pVR1, pCA, andpBHAR.

The gene region of vaccinia virus where the gene encoding a polypeptide having immunogenicity is introduced is a region that is not indispensable for the life cycle of the vaccinia virus. Examples of the region include the hemagglutinin (HA) gene, the thymidine kinase (TK) gene, the B5R gene (region between B4R gene and B6R gene), and the F fragment. For example, in a recombinant having an HA gene into which a gene encoding a polypeptide having immunogenicity is introduced, the HA gene is divided by the foreign gene introduced thereinto to lose the function. As a result, the plaque does not adsorb chicken erythrocytes and therefore looks white. Accordingly, the recombinant can be readily selected. In a recombinant having a TK gene into which a gene encoding a polypeptide having immunogenicity is introduced, the TK gene loses its function. As a result, 5-bromodeoxyuridine (BudR) does not lethally act thereon. Accordingly, the recombinant can be selected with BudR. Furthermore, in a recombinant having a B5R gene into which a gene encoding a polypeptide having immunogenicity is introduced, the plaque of the recombinant is small in size. Accordingly, the recombinant can be selected based on the size of the plaque. It is further desirable that the gene at the foreign gene-introducing region changes the phenotype of the virus by substitution, addition, insertion, and/or deletion of one or more nucleotides to thereby make the selection of the recombinant easy.

The usable cell for infection with the vaccinia virus vector is a cell that can be infected with vaccinia virus, such as a Vero cell, a HeLa cell, a CV1 cell, a COS cell, a RK13 cell, a BHK cell, a primary rabbit kidney cell, a BSC-1 cell, a HTK-143 cell, a Hep2 cell, and a MDCK cell.

In the introduction of a gene encoding a polypeptide having immunogenicity, an appropriate promoter may be operatively linked upstream of the gene encoding a polypeptide having immunogenicity. Any promoter can be used, and examples thereof include an AT1 promoter, PSFJ1-10, PSFJ2-16, ap7.5 promoter, a modified p7.5 promoter (7.5E), ap11K promoter, a T7.10 promoter, a CPX promoter, a HF promoter, a H6 promoter, and a T7 hybrid promoter.

The gene encoding a polypeptide having immunogenicity may be introduced into a vaccinia virus vector by a known method for constructing a recombinant vaccinia virus vector. Such a method can be performed according to description in "Supplement Experimental Medicine, The Protocol Series, Experimental Protocols for Gene Transfer & Expression Analysis (Idenshi Donyu & Hatsugen Kaiseki Jikken-ho), (edited by Izumi Saito, et al., YODOSHA CO., LTD., Sep. 1, 1997)"; "DNA Cloning 4 - Mammalian System -, 2nd ed. (edited by D. M. Glover, et al., translation supervised by Ikunoshin Kato, TaKaRa); "The EMBO Journal, vol. 6, pp. 3379-3384, 1987", for example.

The set of virus vectors for a prime/boost vaccine according to the present invention includes a Sendai virus vector expressively carrying a gene encoding a polypeptide having the immunogenicity. Herein, the term "the immunogenicity" refers to immunogenicity possessed by the polypeptide expressively carried by the vaccinia virus vector according to the present invention. That is, the gene encoding a polypeptide having the immunogenicity expressively carried by the Sendai virus vector according to the present invention may be the same as or different from the gene encoding a polypeptide having immunogenicity carried by the vaccinia virus vector according to the present invention, as long as both the polypeptides have the same immunogenicity.

Sendai virus reproduces itself without interacting with the host genome and is not pathogenic to human and is therefore believed to be highly safe in application to human when used as a vector. The Sendai virus vector in the present invention may have replicability equivalent to that of the wild-type or may be a deficient vector not having replicability. The Sendai virus vector according to the present invention may be a one having modified arrangement of genes or a modified nucleotide sequence of the genome of wild-type Sendai virus. Furthermore, the Sendai virus vector may be derived from a Sendai virus mutant having attenuation mutations or temperature-sensitive mutations in the envelope protein or capsid protein.

The Sendai virus vector expressively carrying a gene encoding a polypeptide having the immunogenicity in the present invention can be produced by a similar method to that of producing the vaccinia virus vector expressively carrying a gene encoding a polypeptide having immunogenicity described above in accordance with the description in Patent Literature 3 by using Sendai virus in place of vaccinia virus and using a cell that can be infected with a Sendai virus, such as an LLC-MK2 cell, a CV1 cell, a BHK cell, or a human-derived cell, as the cell to be infected with the virus vector. Alternatively, the Sendai virus vector can be also produced by directly linking a gene segment, digested with an appropriate restriction enzyme, encoding a polypeptide having immunogenicity to be introduced to the Sendai virus genome having an introduced site recognizable by the same enzyme, and introducing the resulting recombinant Sendai virus genome into a cell that can be infected with a Sendai virus together with appropriate supporting plasmids. The Sendai virus vector defective in the F protein can be produced in accordance with a known method (International Publication Nos. WO2000/70055 and WO2000/70070).

In another embodiment of the set of virus vectors for a prime/boost vaccine according to the present invention, the set of virus vectors for a prime/boost vaccine comprises (a) a vaccinia virus vector expressively carrying a gene encoding a polypeptide having immunogenicity, (b) a Sendai virus vector expressively carrying a gene encoding a polypeptide having the immunogenicity, and (c) a vaccinia virus vector expressively carrying a gene encoding a CD40 ligand non-cleavage mutant.

The vaccinia virus vector expressively carrying a gene encoding CD40Lm in the present invention can be produced by the same method as that of producing the vaccinia virus vector expressively carrying a gene encoding a polypeptide having immunogenicity described above by using a CD40Lm gene in place of the gene encoding a polypeptide having immunogenicity. The promoter inducing expression of CD40Lm in the vaccinia virus vector expressively carrying a gene encoding CD40Lm according to the present invention is preferably a promoter providing a relatively moderate expression amount, such as a p7.5 promoter.

In the set of virus vectors for a prime/boost vaccine according to the present invention, any of the virus vectors may be used for priming and any of the virus vectors may be used for boosting. Preferably, the vector for priming is any of the following (i) to (iii): (i) a vaccinia virus vector expressively carrying a gene encoding a polypeptide having immunogenicity, (ii) a vaccinia virus vector expressively carrying a gene encoding a polypeptide having immunogenicity and a gene encoding CD40Lm, and (iii) a vaccinia virus vector expressively carrying a gene encoding a polypeptide having immunogenicity and a vaccinia virus vector expressively carrying a gene encoding CD40Lm; and the vector for boosting is a Sendai virus vector expressively carrying a gene encoding a polypeptide having the immunogenicity.

It is to be understood to those skilled in the art pertinent to the present invention that the invention shown as, for example, a method of immunizing or activating immunity of a mammal, in particular, human, with a set of virus vectors for a prime/boost vaccine according to the present invention, a method of using the vectors as a vaccine (including the use as a vaccine), a method of using the vector for producing a medicine, or a pharmaceutical composition containing the vectors and a pharmaceutically acceptable excipient is disclosed by the description in the specification, in particular, by the description in the following examples.

The set of virus vectors for a prime/boost vaccine composed of one or two vaccinia virus vectors and a Sendai virus vector according to the present invention will now be described based on examples. The technical scope of the present invention is not limited to the features shown by the following examples.

### Examples

### Example 1

### (1) Production of vaccinia virus vector carrying gene encoding human immunodeficiency virus envelope protein

### [1-1] Production of m8Δ-<high-pro>-env

### <1-1-1> Preparation of env gene

A gene (Accession No. M38429) encoding envelope protein gp160 and gp120 (env) of a human immunodeficiency virus strain HIV-1 JR-CSF was inserted into the AvrII/XhoI site of pJW322 to prepare pJW322-env. Subsequently, sequences, 6751 st to 6757th, 7367th to 7373rd, and 8305th to 8311th, of the env gene, which correspond to transcription terminator sequences of vaccinia virus, were mutated as shown below by in vitro mutagenesis to prepare pJW322-env2 for efficiently expressing the env. These mutations do not change the amino acid sequence of the env.

675 1 st to 6757th
   Before mutation: TTTTTAT (SEQ ID NO: 1)
   After mutation: TTTCTAT (SEQ ID NO: 2)
7367th to 7373rd
   Before mutation: TTTTTCT (SEQ ID NO: 3)
   After mutation: TTCTTCT (SEQ ID NO: 4)
8305th to 8311th
   Before mutation: TTTTTCT (SEQ ID NO: 5)
   After mutation: TTTCTCT (SEQ ID NO: 6)

Subsequently, the env gene was amplified by PCR using pJW322-env2 as the template and the following primers and was isolated.

Forward primer; 5'-TTTCGGACCGCCACCATGAGAGTGAAGGGGATCAGG-3' (SEQ ID NO:7),
Reverse primer; 5'-ATAGGCCGGCCTTATAGCAAAGCCCTTTCCAAGC-3' (SEQ ID NO: 8).

The resulting PCR product was purified and was then digested with restriction enzymes Fsel and RsrII.

### <1-1-2> Gene insertion into vaccinia virus genome

Vaccinia virus strain LC16m8Δ (m8Δ-<high-pro>) (Suzuki H., et al., Vaccine, vol. 27, pp. 966-971, 2009) carrying a genome into which an AT1 promoter, ten contiguous modified p7.5 promoters (7.5Es), and a multi-cloning site (MCS) were inserted was purified by ultracentrifugation using a 20-40% sucrose gradient. The sequence of the AT1 promoter and ten repeating 7.5Es serves as a promoter (high-expression promoter) promoting efficient expression of a gene downstream thereof.

Subsequently, genomic DNA was extracted from the purified m8Δ-<high-pro> by a phenol/chloroform/isoamyl alcohol method and was concentrated by ethanol precipitation. The env gene in (1), [1-1], <1-1-1> of this Example was inserted into the FseI/RsrII site of the genomic DNA to give a genome for a vaccinia virus vector m8Δ-<high-pro>-env carrying the genome containing the high-expression promoter and the env gene.

### <1-1-3> Purification of vaccinia virus containing gene inserted

2.4 ×10⁵ baby hamster kidney cells (BHK cells) were seeded in a 100-mm dish and were cultured overnight. Canarypox virus was added to the medium at a multiplicity of infection (MOI) of 10, followed by culturing at 33°C for 1 hour.

Subsequently, unadsorbed canarypox virus was removed by washing, and then a mixture of lipofectamine LTX plus (Invitrogen Inc.) and the genome for m8Δ-<high-pro>-env in (1), [1-1], <1-1-2> of this Example, prepared in accordance with the attached specification, was added to the dish, followed by culturing overnight. The cultured BHK cells were freeze-thawed. The resulting lysate was diluted and was added to rabbit kidney-derived cells (RK13 cells) cultured in a 24-well plate, followed by culturing at 33°C to form a single plaque. The resulting single plaque was freeze-thawed again to obtain a lysate. The lysate was diluted and was added to RK13 cells cultured in a 24-well plate, followed by culturing at 33°C to form a single plaque. The single plaque was collected as a vaccinia virus vector m8Δ-<high-pro>-env carrying the genome containing the high-expression promoter and the env gene.

### <1-1-4> Confirmation of env expression: ELISA of plaque

The plaque of m8Δ-<high-pro>-env collected in (1), [1-1], <1-1-3> of this Example was fixed in a 2% (w/v) paraformaldehyde/PBS solution, washed with PBS, further blocked with a 5% (w/w) skim milk/PBS solution, and then washed with PBS. Subsequently, the expression of env in the plaque was confirmed by ELISA using an anti-env human antibody as the primary antibody and an alkaline phosphatase-linked anti-human IgG antibody as the secondary antibody, in accordance with a common procedure.

### <1-1-5> Confirmation of env expression: Western blotting

The m8Δ-<high-pro>-env in (1), [1-1], <1-1-3> of this Example was added to RK13 cells at an MOI of 10, followed by adsorption at 33°C for 1 hour. Subsequently, unadsorbed virus was removed by washing, and a medium was added thereto, followed by culturing overnight.

Subsequently, about 1 µg of the RK13 cells were subjected to electrophoresis on a 10% polyacrylamide gel, followed by Western blotting using a serum of an HIV-1 infected subject to confirm the expression of env, in accordance with a common procedure. The results are shown in Figure 2.

### <1-1-6> Mass culture of vaccinia virus vector

The m8Δ-<high-pro>-env in (1), [1-1], <1-1-3> of this Example was mass-cultured with RK13 cells and was then purified and concentrated by ultracentrifugation using a 36% (w/v) sucrose cushion. The virus titer was measured with RK13 cells.

### [1-2] Production of m8Δ-<low-pro>-hCD40Lm

### <1-2-1> Preparation of plasmid

The p7.5 promoter and the hCD40Lm gene were amplified by PCR using pCA-hCD40Lm3 containing a human CD40 ligand non-cleavage mutant (hCD40Lm) gene as the template and the following primers and were isolated. The p7.5 promoter is a vaccinia virus-derived promoter that is commonly used and promotes expression of a gene downstream thereof, but the expression amount of the downstream gene is low compared to the case of the high-expression promoter described above.

Forward primer; 5'-AGTGGATCCGCCAGCATGATCGAAACATACAACCAA-3' (SEQ ID NO: 9),
Reverse primer; 5'-AGACCCGAGTCAGAGTTTGAGTAAGCCAAAGGA-3' (SEQ ID NO: 10).

The resulting PCR product was purified and was then digested with restriction enzymes BamHI and AvaI, followed by insertion into the BamHI/AvaI site of the hemagglutinin (HA) gene of plasmid pVR1 (Shida H., et al., EMBO J., vol. 6, pp. 3379-3384, 1987) to give pVRI-hCD40Lm.

### <1-2-2> Insertion of gene into vaccinia virus genome

BHK cells were cultured in a 60-mm dish until a confluence of 80%, and vaccinia virus strain LC16m8Δ was added thereto at an MOI of 0.05, followed by culturing at 33°C for 1 hour.

Subsequently, a mixture of lipofectamine LTX plus (Invitrogen Inc.) and pVR1 -hCD40Lm in (1), [1-2], <1-2-1> of this Example, prepared in accordance with the attached specification, was added, followed by culturing at 33°C for 24 hours to cause homologous recombination between the hemagglutinin (HA) gene of the genome carried by vaccinia virus strain LC16m8Δ and the p7.5 promoter and the hCD40Lm gene insertion site of pVR1-hCD40Lm.

### <1-2-3> Partial purification of gene-inserted vaccinia virus

The BHK cells in (1), [1-2], <1-2-2> of this Example were freeze-thawed. The resulting lysate was added to RK13 cells, followed by culturing at 33°C for 3 days to form a plaque.

Subsequently, chicken erythrocytes were suspended in PBS containing calcium ions and magnesium ions (Ca²⁺-Mg²⁺-PBS) at a concentration of 0.5-2.0% (w/v) to prepare a solution for a hemadsorption test (HAD test). The medium in which the plaque of the RK13 cells was formed was replaced by the HAD test solution, followed by leaving to stand at room temperature for 1 hour. After washing with Ca²⁺-Mg²⁺-PBS, a colorless plaque not showing agglutination of erythrocytes was collected by scraping.

### <1-2-4> Purification of gene-inserted vaccinia virus

The plaque collected in (1), [1-2], <1-2-3> of this Example was further subjected to the procedure in (1), [1-2], <1-2-3> of this Example twice, and thereby vaccinia virus carrying the genome having the p7.5 promoter and the hCD40Lm gene inserted at the HA gene site was purified as m8Δ-<low-pro>-hCD40Lm.

### <1-2-5> Confirmation of CD40Lm expression: Western blotting

The m8Δ-<low-pro>-hCD40Lm prepared in (1), [1-2], <1-2-4> of this Example was subjected to Western blotting as in the procedure described in (1), [1-1], <1-1-5> of this Example to confirm expression of hCD40Lm, in which the amount of cells used in the electrophoresis was 10 µg instead of 1 µg, and the detection of hCD40Lm was performed using an anti-CD40L mouse monoclonal antibody instead of the HIV-1 infected subject serum. The results are shown in Figure 2.

### <1-2-6> Mass culture of vaccinia virus vector

The m8Δ-<low-pro>-hCD40Lm in (1), <1-2-4> of this Example was mass-cultured, purified, and concentrated, and the virus titer was measured, as in the procedures described in (1), [1-1], <1-1-6> of this Example.

### [1-3] Production ofm8Δ-<high-pro>-env-hCD40Lm

### <1-3-1> Preparation of plasmid

The env gene was amplified by PCR using the pJW322-env2 in (1), [1-1], <1-1-1> of this Example as the template and the following primers and was isolated.

Forward primer; 5'-CTAGAATTCGCCACCATGAGAGTGAAGGGGATCAGGAAG-3' (SEQ ID NO: 11),
Reverse primer; 5'-CGTGAGCTCTTATAGCAAAGCCCTTTCCAAGCC-3' (SEQ ID NO: 12).

The resulting PCR product was purified and was then digested with restriction enzymes EcoRI and SacI.

The p7.5 promoter and the hCD40Lm gene were amplified by PCR using the pVR1-hCD40Lm prepared in (1), [1-2], <1-2-1> of this Example as the template and the following primers and were isolated.

Forward primer; 5'-CTAGAGCTCGCCACCATATACTATATAGTAATACCAATA-3' (SEQ ID NO: 13),
Reverse primer; 5'-GTACCCGGGTCAGAGTTTGAGTAAGCCAAAGG-3' (SEQ ID NO: 14).

The resulting PCR product was purified and was then digested with restriction enzymes SacI and XmaI.

Subsequently, the PCR product of the env gene and the PCR product of the p7.5 promoter and hCD40Lm gene were inserted into the EcoRI/Xmal site of pJW322 to prepare pJW322-env-hCD40Lm.

Subsequently, the region of the env gene, p7.5 promoter, and hCD40Lm gene was amplified by PCR using the pJW322-env-hCD40Lm as the template and the following primers and was isolated.

Forward primer; 5'-TTTCGGACCGCCACCATGAGAGTGAAGGGGATCAGGAAG-3' (SEQ ID NO: 15),
Reverse primer; 5'-AGAGGCCGGCCTCAGAGTTTGAGTAAGCCAAAGGA-3' (SEQ ID NO: 16).

The resulting PCR product was purified and was then digested with restriction enzymes FseI and RsrII.

### <1-3-2> Insertion of gene into vaccinia virus genome

The region of the env gene, p7.5 promoter, and hCD40Lm gene prepared in (1), [1-3], <1-3-1> of this Example was inserted into the FseI/RsrII site of the genomic DNA carried by m8Δ-<high-pro> as in the procedure described in (1), [1-1], <1-1-2> in this Example to give a genome for a vaccinia virus vector m8Δ-<high-pro>-env-hCD40Lm carrying the genome containing the high-expression promoter, env gene, and hCD40Lm gene.

### <1-3-3> Purification of gene-inserted vaccinia virus

The vaccinia virus vector m8Δ-<high-pro>-env-hCD40Lm carrying the genome containing the high-expression promoter, env gene, and hCD40Lm gene was purified as in the procedure described in (1), [1-1], <1-1-3> of this Example.

### <1-3-4> Confirmation of env expression: ELISA and Western blotting of plaque

The m8Δ-<high-pro>-env-hCD40Lm in (1), [1-3], <1-3-3> of this Example was subjected to ELISA as in the procedure described in (1), [1-1], <1-1-4> of this Example to confirm the expression of env in the plaque. The m8Δ-<high-pro>-env-hCD40Lm in (1), [1-3], <1-3-3> of this Example was also subjected to Western blotting as in the procedures described in (1), [1-1], <1-1-5> and (1), [1-2], <1-2-5> of this Example to confirm the expression of env and hCD40Lm. The results are shown in Figure 2.

### <1-3-5> Mass culture of vaccinia virus vector

The m8Δ-<high-pro>-env-hCD40Lm in (1), [1-3], <1-3-3> of this Example was mass-cultured, purified, and concentrated, and the virus titer was measured, as in the procedures described in (1), [1-1], <1-1-6> of this Example.

### [1-4] Production ofm8Δ-<high-pro>-hCD40Lm

### <1-4-1> Preparation of plasmid

The hCD40Lm gene was amplified by PCR using the pCA-hCD40Lm3 inserted with the hCD40Lm gene as the template and the following primers and was isolated.

Forward primer; 5'-AAACCCGGGCATGATCGAAACATACAACCAAA-3' (SEQ ID NO: 17),
Reverse primer; 5'-CCATCTAGATCCTCAGAGTTTGAGTAAGCCA-3' (SEQ ID NO: 18).

The resulting PCR product was purified and digested with restriction enzymes Xmal and NotI and was inserted into the XmaI/NotI site of pBHAR having an AT1 promoter and ten contiguous 7.5Es (high-expression promoter) (Jin N-Y, et al., Arch. Virol., vol. 138, pp. 315-330, 1994) to give pBHAR-hCD40Lm.

### <1-4-2> Insertion of gene into vaccinia virus genome

The region of the high-expression promoter and hCD40Lm gene in (1), [1-4], <1-4-1> of this Example was inserted into the genome carried by vaccinia virus strain LC16m8Δ as in the procedure described in (1), [1-2], <1-2-2> of this Example.

### <1-4-3> Purification of gene-inserted vaccinia virus

The vaccinia virus vector m8Δ-<high-pro>-hCD40Lm carrying the genome containing the high-expression promoter and hCD40Lm gene was purified as in the procedures described in (1), [1-2], <1-2-3> and <1-2-4> of this Example.

### <1-4-4> Confirmation of hCD40Lm expression: Western blotting

The m8Δ-<high-pro>-hCD40Lm in (1), [1-4], <1-4-3> of this Example was subjected to Western blotting as in the procedure described in (1), [1-2], <1-2-5> of this Example to confirm the expression of hCD40Lm. The results are shown in Figure 2.

The results shown in Figure 2 demonstrate that the expression amount of hCD40Lm in the cells infected with m8Δ-<high-pro>-hCD40Lm is higher than those of hCD40Lm in cells infected with m8Δ-<high-pro>-env-hCD40Lm and cells infected with m8Δ-<low-pro>-hCD40Lm.

### <1-4-5> Mass culture of vaccinia virus vector

The m8Δ-<high-pro>-hCD40Lm in (1), [1-4], <1-4-3> of this Example was mass-cultured, purified, and concentrated, and the virus titer was measured, as in the procedures described in (1), [1-1], <1-1-6> of this Example.

Figure 1 shows the structures of the genes and promoters inserted into m8Δ-<high-pro>-env, m8Δ-<low-pro>-hCD40Lm, m8Δ-<high-pro>-env-hCD40Lm, and m8Δ-<high-pro>-hCD40Lm in (1) of this Example.

### (2) Production of Sendai virus vector carrying env (SeV-env)

### [2-1] Preparation of plasmid

An env gene having a NotI-recognizing sequence on each end was inserted into the NotI site of pBluescript to give pBluescript-env.

The env gene has A and T contiguous sequences, which are transcription terminator sequences of gene expression of Sendai virus, at three sites. Accordingly, the env gene was subjected to PCR using the following primers to introduce mutations into the contiguous sequences to give pBluescript-env-mut carrying the env gene having the mutations (env-mut gene).

Primer used for mutation
Mutation 1:
Forward primer; 5'-CCATCGTCTTCACTCACTCCTCAGGAGGGGATCCAGAAATTG-3' (SEQ ID NO: 19)

Reverse primer; 5'-
GAATAACACTTTAAAACAGATAGTTGAGAAGCTCCGCGAGCAGTTCAACAACAAGACC
ATCGTCTTCACTCACTCCTCAGGAG-3' (SEQ ID NO: 20)

Mutation 2:

Forward primer; 5'-GTGAAGATCGAACCATTAGGAGTAGCACCCACCAAGGCAAAG-
3' (SEQ ID NO: 21)

Reverse primer; 5'-
GAGACATGAGGGACAATTGGAGAAGTGAGCTCTACAAGTACAAGGTCGTGAAGATCGA
ACCATTAGGAGTA-3' (SEQ ID NO: 22)

Mutation 3

Forward primer; 5'-
CGCATCGTGTTCTCTGTACTTTCTATAGTGAATAGAGTTAGGCAGG-3' (SEQ ID NO: 23)

Reverse primer; 5'-
GTTTGACATAACAAAATGGCTGTGGTACATCAAGATCTTCATCATGATCGTGGAGGCC
TGATCGGTCTCCGCATCGTGTTCTCTGTACTTTCTATAG-3' (SEQ ID NO: 24)

### [2-2] Insertion of gene into Sendai virus genome

The pBluescript-env-mut prepared in (2), [2-1] of this Example was digested with NotI to cut out the env-mut gene segment. This gene segment was inserted into the NotI site of plasmid pSeV/ΔF containing the Sendai virus genome having a Notl-recognizing sequence on the 3' end thereof but not containing the gene (F) encoding the Sendai virus surface protein, fusion, to give pSeV-env-mut/ΔF.

### [2-3] Purification of gene-inserted Sendai virus

293T cells were transfected with a mixture of pSeV-env-mut/ΔF in (2), [2-2] of this Example and supporting plasmids, pCAGGS-NP, pCAGGS-P, pCAGGS-L, and pCAGGS-T7, followed by culturing.

Subsequently, the culture supernatant of 293T cells was added to F-expressing cells, LLC-MK2/F/Ad cells, followed by culturing. The culture supernatant was collected.

Subsequently, the collected culture supernatant was subjected to limiting dilution and infection to LLC-MK2/F/Ad cells using a 96-well plate to clone a virus having pSeV-env-mut/ΔF.

The cloned virus was used as a Sendai virus vector SeV-env carrying a genome containing the env gene. The nucleotide sequence of the env gene carried by SeV-env was confirmed to have a mutation of A at the position 450 to G, resulting in a mutation of asparagine to aspartic acid in the amino acid sequence. The SeV-env was proliferated by infecting to LLC-MK2/F/Ad cells.

### [2-4] Mass culture of Sendai virus vector

The SeV-env in (2), [2-3] of this Example was added to 36 flasks each having a culture area of 225 cm² in which LLC-MK2/F/Ad cells were cultured, followed by culturing for 24 hours. The medium was replaced by fresh medium, followed by culturing for further 48 hours. The culture supernatant was then collected, filtered, and concentrated using an ultrafiltration filter.

### (3) Production of plasmid carrying env gene (DNA-env)

### [3-1] Insertion of gene into plasmid

The env gene was amplified by PCR using pJW322-env2 in (1), [1-1], <1-1-1> of this Example as the template and the following primers and was isolated.

Forward primer; 5'-CTAGAATTCGGCATCTCCTATGGCAGGAAGAAG-3' (SEQ ID NO: 25),
Reverse primer; 5'-CGTGAATTCACCCATCTTATAGCAAAGCCCTT-3' (SEQ ID NO: 26).

The resulting PCR product was purified and was then digested with restriction enzyme EcoRI and was inserted into the EcoRI site of mammalian cell expression vector plasmid pCAGGS to give pCAGGS plasmid carrying the env gene (DNA-env).

### [3-2] Confirmation of env expression

The DNA-env in (3), [3-1] of this Example was mixed with polyethylene imine (PEI: Polysciences Inc.), and the mixture was transfected into 293T cells. The cells were cultured for 2 days and were subjected to Western blotting as in the procedure described in (1), [1-1], <1-1-5> of this Example to confirm expression of env.

### [3-3] Mass culture of DNA-env

The DNA-env in (3), [3-1] of this Example was transformed into Escherichia coli XL1-blue and mass-cultured and was then purified using EndoFree Plasmid Purification (Qiagen, Inc.).

### Example 2

### Confirmation of effect of activating cellular immunity: Vaccination with coexpression vaccinia virus vector in priming with DNA-env/boosting with vaccinia virus vector

### (1) Primary immunization (priming)

The DNA-env in (3), [3-3] of Example 1 was dissolved in PBS at a concentration of 1 µg/mL to prepare a DNA-env solution. Nine C57BL/6 mice were each intramuscularly injected (priming) with 50 µL (50 µg) of this solution in accordance with a common method and were bred for 2 weeks. Subsequently, the mice were each intramuscularly injected (priming) with 50 µL (50 µg) of the DNA-env solution again in accordance with a common method and were bred for 8 weeks.

### (2) Booster immunization (boosting)

The m8Δ-<high-pro> in (1), [1-1], <1-1-2> of Example 1, the m8Δ-<high-pro>-env in (1), [1-1], <1-1-6> of Example 1, and the m8Δ-<high-pro>-env-hCD40Lm in (1), [1-3], <1-3-5> of Example 1 were each dissolved in PBS at 1×10⁸ PFU/mL to prepare a m8Δ-<high-pro> solution, a m8Δ-<high-pro>-env solution, and a m8Δ-<high-pro>-env-hCD40Lm solution, respectively.

The mice in (1) of this Example were divided into three groups, control group, group A, and group B, each consisting of three mice. The mice in the control group, the mice in group A, and the mice in group B were intradermally injected (boosting) with 100 µL (1×10⁷ PFU) of the m8Δ-<high-pro> solution, the m8Δ-<high-pro>-env solution, and the m8Δ-<high-pro>-env-hCD40Lm solution, respectively, in accordance with a common method and were then bred for 2 weeks.

### (3) Extraction of T cells

The spleen was extracted from each mouse in each group in (2) of this Example, and spleen cells were harvested in accordance with a common method. The harvested spleen cells were suspended in an RPMI1640 medium and were centrifuged at 200xg at room temperature for 10 minutes. The supernatant was removed. A 0.8% (w/v) aqueous ammonium chloride solution was added to the cells for hemolysis to remove erythrocytes. The remaining spleen cells were suspended in an RPMI1640 medium and were passed through a nylon mesh to concentrate the T cells, followed by counting the number of cells in accordance with a common method.

### (4) Intracellular cytokine staining

The T cells in (3) of this Example were stimulated with HIV-1 Consensus Subtype B Env (15-mer) Peptides (AIDS Research and Reference Reagent Program) in accordance with the attached specification. Subsequently, CD8-positive IFN-γ-producing cells among the T cells were stained using APC-labeled anti-mouse IFN-γ (eBioscience Company) and PE-labeled anti-mouse CD8 (eBioscience Company) as labeled antibodies and Fixation and Permeabilization Solution Kit with BD GolgiStop (Becton, Dickinson and Company) in accordance with the attached specifications.

### (5) Counting the number of stained cells by FACS

The number of CD8-positive IFN-γ-producing cells stained in (4) of this Example was measured using FACS CantoII (Becton, Dickinson and Company). The average value of measurement results in each group was determined and was expressed in a graph. The results are shown in Figure 3.

As shown in Figure 3, the average values were about 1.25% and about 1% in group A and group B, respectively, whereas no CD8-positive IFN-γ-producing cell was detected in control group.

These results revealed that cellular immunity is activated by priming with an antigen protein expressing plasmid and then boosting with an antigen protein expressing vaccinia virus vector. In addition, it was revealed that the effect of activating cellular immunity is hardly enhanced by using a vector coexpressing CD40Lm in addition to the antigen protein as the vaccinia virus vector in this case.

### Example 3

### Confirmation of effect of activating cellular immunity: Scarification vaccination with coexpression vaccinia virus vector in priming with DNA-env/boosting with vaccinia virus vector

### (1) Priming and boosting

Two C57BL/6 mice, mouse A and mouse B, were each primed and boosted as in the procedures described in (1) and (2) of Example 2, in which the m8Δ-<high-pro>-env-hCD40Lm in (1), [1-3], <1-3-5> of Example 1 was dissolved in PBS at a concentration of 1×10⁹ PFU/mL to prepare a m8Δ-<high-pro>-env-hCD40Lm solution, and mouse A was boosted with 10 µL (1×10⁷ PFU) of this solution by scarification vaccination using a bifurcated needle; and mouse B was boosted with 100 µL (1×10⁷ PFU) of the 1×10⁸ PFU/mL m8Δ-<high-pro>-env-hCD40Lm solution in (2) of Example 2 by intradermal injection.

### (2) Extraction of T cells, intracellular cytokine staining, and counting the number of stained cells by FACS

Mouse A and mouse B in (1) of this Example were subjected to extraction of T cells, intracellular cytokine staining, and counting the number of stained cells by FACS as in the procedures described in (3) to (5) of Example 2. The results are shown in Figure 4.

As shown in Figure 4, the ratio of the CD8-positive IFN-γ-producing cells in mouse A was about 3.25%, whereas the ratio in mouse B was about 1.25%.

The results revealed that in activation of cellular immunity by priming with an antigen protein expressing plasmid and then boosting with an antigen protein/hCD40Lm coexpressing vaccinia virus vector, the effect of activating cellular immunity is enhanced by boosting vaccination by scarification using a bifurcated needle instead of intradermal injection.

### Example 4

### Confirmation of effect of activating humoral immunity: Comparison between priming with DNA-env/boosting with vaccinia virus vector and priming with vaccinia virus vector/boosting with Sendai virus vector

### (1) Priming

Two C57BL/6 mice, mouse A and mouse B, were primed (priming); mouse A was primed as in the procedure described in (1) of Example 2, and mouse B was vaccinated with 10 µL (1×10⁷ PFU) of a m8Δ-<high-pro>-env solution prepared by dissolving the m8Δ-<high-pro>-env in (1), [1- 1], <1-1-6> of Example 1 in PBS at a concentration of 1×10⁹ PFU/mL by scarification using a bifurcated needle, and the mice were bred for 8 weeks.

### (2) Boosting

Mouse A in (1) of this Example was vaccinated (boosting) by scarification using a bifurcated needle with 10µL (1×10⁷ PFU) of the m8Δ-<high-pro>-env solution in (1) of this Example, and was bred for 2 weeks. On the other hand, mouse B in (1) of this Example was vaccinated (boosting) by nasal injection with 10 µL (4×10⁷ CFU) of a SeV-env solution prepared by dissolving the SeV-env in (2), [2-4] of Example 1 in PBS at a concentration of 4×10⁹ CFU/mL, and was bred for 2 weeks.

### (3) Extraction of serum

Blood was collected from the mouse A and mouse B in (2) of this Example in accordance with a common method, and serum was isolated.

### (4) ELISA

### [4-1] Preparation of env-immobilized plate

A TMN buffer solution containing 10 mmol/L of Tris-HCl (pH 7.4), 3 mmol/L of MgCl₂, and 0.5% (v/v) of NP40 was prepared. The DNA-env in (3), [3-3] of Example 1 was transfected into 293T cells cultured until a confluence of 80% in a 100-mm dish, followed by culturing 2 days. The 293T cells were dissolved in the TMN buffer solution and were ultrafiltrated to prepare a protein solution not containing proteins having a molecular weight of smaller than 100 kDa. The resulting solution was added to a 96-well plate for ELISA, followed by incubation to give an env-immobilized plate onto which antigen proteins containing env were immobilized.

### [4-2] ELISA using env-immobilized plate

ELISA was performed using the serum in (3) of this Example diluted to 100-fold (1/100), 300-fold (1/300), 900-fold (1/900), or 2700-fold (1/2700) or HIV-1 infected subject serum (positive control) as the primary antibody, a horseradish peroxidase-linked anti-rat IgG antibody or a horseradish peroxidase-linked anti-human IgG antibody as the secondary antibody and TMB ELISA Substrate Solution (eBioscience Company) as the coloring reagent, in accordance with a common method, and the absorbance was measured at a wavelength of 450 nm. The results are shown in Figure 5.

As shown in the graph at the left in Figure 5, the absorbance values in mouse A were 0 at any of 1/100, 1/300, 1/900, and 1/2700, which results show that no binding affinity of an anti-env antibody was recognized. On the other hand, as shown in the graph at the right in Figure 5, the absorbance values in mouse B were about 2.4 at 1/100 and 1/300, about 2.25 at 1/900, and about 1.5 at 1/2700, which results show that the binding affinity of an anti-env antibody was recognized.

These results revealed that humoral immunity is activated by priming with an antigen protein expressing vaccinia virus vector and then boosting with an antigen protein expressing Sendai virus vector, whereas humoral immunity is not activated by priming with an antigen protein expressing plasmid and then boosting with an antigen protein expressing vaccinia virus vector.

### Example 5

### Confirmation of immunostimulating effect: Vaccination with coexpressing vector in priming with DNA-env/boosting with vaccinia virus vector

### (1) Priming

Fifteen C57BL/6 mice were divided into three groups, control group, group A, and group B, each consisting of five mice. The mice in group A and the mice in group B were vaccinated (priming) with 10 µL (1×10⁷ PFU) of the m8Δ-<high-pro>-env solution in (1) of Example 4 and the m8Δ-<high-pro>-env-hCD40Lm solution in (1) of Example 3, respectively, by scarification using a bifurcated needle and were then bred for 8 weeks. The mice in control group were not vaccinated.

### (2) Boosting

The mice in groups A and B in (1) of this Example were each vaccinated (boosting) with 10 µL (4×10⁷ CFU) of the SeV-env solution in (2) of Example 4 by nasal injection, and were bred for 2 weeks.

### (3) Extraction of T cells and serum

Serum was collected from each mouse in each group in (2) of this Example, and T cells were extracted as in the procedure described in (3) of Example 2.

### (4) Intracellular cytokine staining and counting the number of stained cells by FACS

The T cells collected in (3) of this Example were subjected to intracellular cytokine staining and counting the number of stained cells by FACS as in the procedures described in (4) and (5) of Example 2. The measurement results were subjected to statistical examination between group A and group B. The results are shown in Figure 6.

As shown in Figure 6, CD8-positive IFN-γ-producing cells were not detected in control group, and the average ratios of the CD8-positive IFN-γ-producing cells in group A and group B were about 7.2% and about 6%, respectively. There was no significant difference between the measurement values in group A and group B.

These results revealed that cellular immunity is activated by priming with an antigen protein expressing vaccinia virus vector or an antigen protein/hCD40Lm coexpressing vaccinia virus vector and then boosting with an antigen protein expressing Sendai virus vector. In addition, it was revealed that the effect of activating cellular immunity is not enhanced by using an antigen protein/CD40Lm coexpressing vector as the vaccinia virus vector in this case.

### (5) ELISA

The sera of group A and the sera of group B collected in (3) of this Example were subjected to ELISA as in the procedure described in (4) of Example 4. In addition, the average value of the results of each group was determined and is shown in the graph at the left in Figure 7.

As shown in the graph at the left in Figure 7, the absorbance values in group A were about 2.4 at 1/100 and 1/300, about 2.2 at 1/900, and about 1.4 at 1/2700. Similarly, the absorbance values in group B were about 2.4 at 1/100 and 1/300, about 2.3 at 1/900, and about 1.95 at 1/2700. Accordingly, in both group A and group B, binding affinity of anti-env antibodies was recognized. In addition, it was confirmed that the binding affinity of anti-env antibodies of group B is higher than that of group A.

### (6) TZM-bl assay

The sera of each group collected in (3) of this Example were subjected to TZM-bl assay in accordance with a known method (J. Virol., vol. 79, pp. 10108-10125, 2005) to measure the neutralizing activity of the anti-env antibodies contained in the serum. Specifically, 3.3 µg of pCAGGS-SF162env and 6.6 µg of plasmid pSG3-ΔEnv having an env gene-deficient HIV-1 genome were transfected into 293T cells cultured until a confluence of 80% in a 100-mm dish, followed by culturing for 48 hours. The supernatant was collected to give a pseudotyped virus solution containing pseudotyped virus covered with an env envelope. This virus solution was passed through a filter of 0.45 µm and was stored at ?80°C.

Subsequently, five-fold serial dilution of the pseudotyped virus solution was added to the TZM-bl cells cultured in a 96-well plate, followed by culturing for 48 hours. The luminescence of luciferase was measured using Bright Glo reagent (Promega Corporation) to determine the TCID₅₀ of the pseudotyped virus solution.

Subsequently, the serial dilution of the serum collected in (3) of this Example was prepared, and a pseudotyped virus solution of 200 TCID₅₀ was added to each diluted serum to prepare 100 µL of each mixture solution, followed by incubation at 37°C for 1 hour. The prepared mixture solution was added to the TZM-bl cells cultured until a confluence of 80% in a 96-well plate, followed by culturing for 72 hours. The luminescence of luciferase was then measured using Bright Glo reagent (Promega Corporation) to determine the dilution multiple (ID₅₀) of serum that inhibits 50% of TZM-bl cells from being infected with the pseudotyped virus. The results are shown in the table at the right in Figure 7.

As shown in the table at the right of Figure 7, the average ID₅₀ in group B was 8022, whereas the average ID₅₀ in group A was 300. Thus, it was confirmed that the neutralizing activity of anti-env antibodies in group B is considerably higher than that in group A.

These results revealed that humoral immunity is activated by priming with an antigen protein expressing vaccinia virus vector or an antigen protein/hCD40Lm coexpressing vaccinia virus vector and boosting with an antigen protein expressing Sendai virus vector. In addition, it was revealed that the activation of humoral immunity is enhanced by using an antigen protein/CD40Lm coexpressing vector as the vaccinia virus vector in this case.

### Example 6

### Confirmation of immunostimulating effect: Mixed vaccination with virus vectors in priming with DNA-env/boosting with vaccinia virus vector

### (1) Priming

Nine C57BL/6 mice were primed as in the procedure described in (1) of Example 2.

### (2) Boosting

The mice in (1) in this Example were divided into three groups, group A, group B, and control group, each consisting of three mice. The m8Δ-<low-pro>-hCD40Lm in (1), [1-2], <1-2-6> of Example 1, the m8Δ-<high-pro>-hCD40Lm in (1), [1-4], <1-4-5> of Example 1, and the m8Δ-<high-pro> in (1), [1-1], <1-1-2> of Example 1 were each dissolved in PBS at a concentration of 1×10⁹ PFU/mL to prepare a m8Δ-<low-pro>-hCD40Lm solution, a m8Δ-<high-pro>-hCD40Lm solution, and a m8Δ-<high-pro> solution, respectively.

The mice in group A, group B, and control group were each vaccinated (boosting) by scarification using a bifurcated needle with 10 µL (1×10⁷ PFU) of a mixture in a combination shown below of the m8Δ-<low-pro>-hCD40Lm solution, the m8Δ-<high-pro>-hCD40Lm solution, and the m8Δ-<high-pro> solution prepared in (2) of this Example and the m8Δ-<high-pro>-env solution in (1) of Example 4, and were bred for 2 weeks.

Group A: m8Δ-<high-pro>-env solution and m8Δ-<low-pro>-hCD40Lm solution,
Group B: m8Δ-<high-pro>-env solution and m8Δ-<high-pro>-hCD40Lm solution, and
Control group: m8Δ-<high-pro>-env solution and m8Δ-<high-pro> solution.

### (3) Extraction of T cells and serum

Serum was collected from each mouse in each group in (2) of this Example in accordance with a common method, and T cells were extracted as in the procedure described in (3) of Example 2.

### (4) Intracellular cytokine staining and counting the number of stained cells by FACS

The T cells collected in (3) of this Example were subjected to intracellular cytokine staining and counting the number of stained cells by FACS as in the procedures described in (4) and (5) of Example 2. The results are shown in Figure 8.

As shown in Figure 8, the average ratios of the CD8-positive IFN-γ-producing cells in group A, group B, and control group were about 7%, about 3.5%, and about 3.2%, respectively.

These results revealed that in activation of cellular immunity through priming with an antigen protein expressing plasmid and then boosting with an antigen protein expressing vaccinia virus vector, the activation of cellular immunity is enhanced by performing the boosting by vaccination with a mixture of an antigen protein expressing vaccinia virus vector and a CD40Lm expressing vaccinia virus vector. In addition, it was revealed that the cellular immunity is enhanced when the expression amount of CD40Lm is relatively low, but is not enhanced when the expression amount of CD40Lm is relatively high.

### (5) ELISA

The sera collected in (3) of this Example were subjected to ELISA as in the procedure described in (4) of Example 4. Typical results of the sera of group A are shown in Figure 9.

As shown by the typical results in Figure 9, the absorbance values in group A were 0 at any dilution rate, which show that no binding affinity of anti-env antibodies was recognized. Similarly, no binding affinity of anti-env antibodies was recognized also in group B and control group (not shown in the figure).

### (6) TZM-bl assay

The sera collected in (3) of this Example were subjected to TZM-bl assay as in the procedure described in (6) of Example 5. As a result, no neutralizing activity of anti-env antibodies was recognized in the sera of group A, group B, and control group (not shown in the figure).

These results revealed that humoral immunity is not activated by priming with an antigen protein expressing plasmid and then boosting with a mixture of an antigen protein expressing vaccinia virus vector and a CD40Lm expressing vaccinia virus vector.

### Example 7

### Confirmation of immunostimulating effect: Mixed vaccination with virus vectors in priming with vaccinia virus vector/boosting with Sendai virus vector

### (1) Priming

Fifteen C57BL/6 mice were divided into three groups, control group, group A, and group B, each consisting of five mice. The mice in group A and group B were vaccinated (priming) by scarification using a bifurcated needle with 10 µL (1×10⁷ PFU) of a mixture in a combination shown below of the m8Δ-<high-pro>-env solution in (1) of Example 4, the m8Δ-<high-pro> solution in (2) of Example 6, and the m8Δ-<low-pro>-hCD40Lm solution in (2) of Example 6, and were bred for 8 weeks. The mice in control group were not vaccinated.

Group A: m8Δ-<high-pro>-env solution and m8Δ-<low-pro>-hCD40Lm solution, and
Group B: m8Δ-<high-pro>-env solution and m8Δ-<high-pro> solution.

### (2) Boosting

The mice in groups A and B in (1) of this Example were each vaccinated (boosting) with 10 µL (4×10⁷ CFU) of the SeV-env solution in (2) of Example 4 by nasal injection, and were bred for 2 weeks.

### (3) Extraction of T cells and serum

Serum was collected from each mouse in each group in (1) of this Example, and T cells were extracted as in the procedure described in (3) of Example 2.

### (4) Intracellular cytokine staining and counting the number of stained cells by FACS

The T cells collected in (3) of this Example were subjected to intracellular cytokine staining and counting the number of stained cells by FACS as in the procedures described in (4) and (5) of Example 2. The measurement results were subjected to statistical tests between group A and group B. The results are shown in Figure 10.

As shown in Figure 10, the average ratios of the CD8-positive IFN-γ-producing cells in group A and group B were about 12% and about 6%, respectively, but no CD8-positive IFN-γ-producing cell was detected in control group. The measurement value in group A was confirmed to be significantly larger than the measurement value in group B.

These results revealed that in activation of cellular immunity through priming with an antigen protein expressing vaccinia virus vector and then boosting with an antigen protein expressing Sendai virus vector, the activation of cellular immunity is enhanced by performing the priming by vaccination with a mixture of an antigen protein expressing vaccinia virus vector and a CD40Lm expressing vaccinia virus vector.

### (5) ELISA

The sera of group A and the sera of group B collected in (3) of this Example were subjected to ELISA as in the procedure described in (4) of Example 4. In addition, the average value of the results of each group was determined and is shown in the graph at the left in Figure 11.

As shown in the graph at the left in Figure 11, the absorbance values in group A were about 2.4 at 1/100 and 1/300, about 2.25 at 1/900, and about 1.6 at 1/2700. On the other hand, the absorbance values in group B were about 2.3 at 1/100, about 2.1 at 1/300, about 1.6 at 1/900, and about 0.75 at 1/2700. Accordingly, it was confirmed that the binding affinity of anti-env antibodies in group A is higher than that in group B.

### (6) TZM-bl assay

The sera of group A and the sera of group B collected in (3) of this Example were subjected to TZM-bl assay as in the procedure described in (6) of Example 5. In addition, the average value of the results of each group was determined and is shown in the table at the right in Figure 11.

As shown in the table at the right of Figure 11, the average ID₅₀ in group B was 1581.6, whereas the average ID₅₀ in group A was 1542.6. Thus, it was confirmed that the neutralizing activity levels of anti-env antibodies in group A and group B are substantially the same.

These results revealed that in activation of humoral immunity through priming with an antigen protein expressing vaccinia virus vector and then boosting with an antigen protein expressing Sendai virus vector, the activation of humoral immunity is enhanced by performing the priming through vaccination with a mixture of an antigen protein expressing vaccinia virus vector and a CD40Lm expressing vaccinia virus vector.

### Example 8

### Confirmation of immunostimulating effect: Vaccination with coexpressing vector and mixed vaccination with virus vectors in priming with vaccinia virus vector/boosting with Sendai virus vector

### (1) Priming

Twenty-five C57BL/6 mice were divided into five groups, control group, group A, group B, group C, and group D, each consisting of five mice. The mice in groups A, B, C, and D were vaccinated (priming) by scarification using a bifurcated needle with 10 µL (1×10⁷ PFU) of a mixture in a combination shown below of the m8Δ-<high-pro>-env solution in (1) of Example 4, the m8Δ-<high-pro> solution in (2) of Example 6, the m8Δ-<high-pro>-env-hCD40Lm solution in (1) of Example 3, and the m8Δ-<low-pro>-hCD40Lm solution in (2) of Example 6, and were bred for 8 weeks. The mice in control group were not vaccinated.

Group A: m8Δ-<high-pro>-env solution and m8Δ-<low-pro>-hCD40Lm,
Group B: m8Δ-<high-pro>-env solution and m8Δ-<high-pro>,
Group C: m8Δ-<high-pro>-env-hCD40Lm solution, and
Group D: m8Δ-<high-pro>-env solution.

### (2) Boosting

The mice in groups A, B, C, and D in (1), [1-1] of this Example were each vaccinated (boosting) with 10 µL (4×10⁷ CFU) of the SeV-env solution in (2) of Example 4 by nasal injection, and were bred for 2 weeks.

### (3) Extraction of T cells and serum

Serum was collected from each mouse in each group in (1), [1-1] of this Example, and T cells were extracted as in the procedure described in (3) of Example 2.

### (4) Intracellular cytokine staining and counting the number of stained cells by FACS

The T cells in each group collected in [1-3] of this Example were divided into two groups. One of the two groups was subjected to staining of CD4-positive IFN-γ-producing cells using APC-labeled anti-mouse IFN-γ (eBioscience Company) and V450 Rat anti-mouse CD4 (Becton, Dickinson and Company) as labeled antibodies as in the procedure described in (4) of Example 2. The other group was subjected to staining of CD4-positive IL-4-producing cells using PE-Cy7 Rat anti-mouse IL-4 (Becton, Dickinson and Company) and V450 Rat anti-mouse CD4 (Becton, Dickinson and Company) as labeled antibodies as in the procedure described in (4) of Example 2.

Subsequently, stained cells were counted by FACS as in the procedure described in (5) of Example 2. The measurement results of CD4-positive IFN-γ-producing cells were subjected to statistical examination between group A and group B and between group C and group D; and the measurement results of CD4-positive IL-4-producing cells were subjected to statistical examination between each group and control group. The results are shown in Figure 12.

As shown in the graph at the left in Figure 12, though the number of CD4-positive IFN-γ-producing cells was not detected in control group, the average ratios of the CD4-positive IFN-γ-producing cells detected in groups A, B, C, and D were about 0.2%, about 0.4%, about 0.22%, and about 0.35%, respectively. As shown in the graph at the right in Figure 12, the average fluorescent intensities of CD4-positive IL-4-producing cells in groups A, B, C, D, and control were about 23, about 19, about 29, about 17, and about 10, respectively.

These results revealed that in priming with an antigen protein expressing vaccinia virus vector and then boosting with an antigen protein expressing Sendai virus vector, expression of CD40Lm through mixed vaccination or coexpression in the priming causes a reduction in the number of CD4-positive IFN-γ-producing cells and a slight increase in the number of CD4-positive IL-4-producing cells, compared with the case of not expressing CD40Lm.

## Claims

1. A set of virus vectors for a prime/boost vaccine, comprising the following virus vector (a) and virus vector (b):
(a) a vaccinia virus vector expressively carrying a gene encoding a polypeptide having immunogenicity; and
(b) a Sendai virus vector expressively carrying a gene encoding a polypeptide having the immunogenicity.

2. The set of virus vectors for a prime/boost vaccine according to Claim 1, further comprising the following virus vector (c):
(c) a vaccinia virus vector expressively carrying a gene encoding a CD40 ligand non-cleavage mutant.

3. The set of virus vectors for a prime/boost vaccine according to Claim 1, wherein the vaccinia virus vector expressively carrying a gene encoding a polypeptide having immunogenicity is a vaccinia virus vector expressively carrying a gene encoding a polypeptide having immunogenicity and a gene encoding a CD40 ligand non-cleavage mutant.

4. The set of virus vectors for a prime/boost vaccine according to any one of Claims 1 to 3, wherein the virus vector (a) or the virus vector (a) and the virus vector (c) are for priming; and the virus vector (b) is for boosting.

5. The set of virus vectors for a prime/boost vaccine according to any one of Claims 1 to 4, wherein the vaccinia virus vector is a vaccinia virus strain LC16, strain LC16m8, or strain Lc16mO and having substitution, addition, insertion, and/or deletion of one or more nucleotides in its B5R gene not to produce any B5R gene product having a normal function.

6. The set of virus vectors for a prime/boost vaccine according to any one of Claims 1 to 5, wherein the polypeptide having immunogenicity is an antigen protein of a microorganism pathogenic to human or a partial peptide thereof or is a human tumor antigen protein or its partial peptide.

7. The set of virus vectors for a prime/boost vaccine according to Claim 6, wherein the pathogenic microorganism is one selected from the group consisting of human immunodeficiency viruses, influenza viruses, human hepatitis viruses, human papillomaviruses, herpes viruses, flaviviruses, severe acute respiratory syndrome viruses, Japanese encephalitis viruses, measles viruses, rubella viruses, mumps viruses, yellow fever viruses, rabies viruses, Ebola viruses, Lassa viruses, polio viruses, St. Louis encephalitis viruses, cholera vibrios, tubercle bacilli, diphtheria bacilli, typhoid bacilli, Whooping cough bacilli, meningococci, tetanus bacilli, mycobacteria, and malaria parasites.
